# EUROPÄISCHE PATENTSCHRIFT

(11) **EP 1 451 150 B1**
(45) Veröffentlichungstag und Bekanntmachung des Hinweises auf die Patenterteilung: **13.01.2010**
(21) Anmeldenummer: 02795066.6
(22) Anmeldetag: 19.11.2002
(51) Int. Cl.: C07C 263/10

(54) **HERSTELLUNG VON ISOCYANATEN IN DER GASPHASE**
PRODUCTION OF ISOCYANATES IN THE GASEOUS PHASE
PRODUCTION D'ISOCYANATES EN PHASE GAZEUSE

(30) Priorität: 28.11.2001 DE 10158160
(43) Veröffentlichungstag der Anmeldung: 01.09.2004
(73) Patentinhaber: BASF SE, 67056 Ludwigshafen (DE)
(72) Erfinder: WÖLFERT, Andreas, 74906 Bad Rappenau (DE); MÜLLER, Christian, 68167 Mannheim (DE); STRÖFER, Eckhard, 68163 Mannheim (DE); PFEFFINGER, Joachim, 67056 Ludwigshafen (DE); WEBER, Markus, 67061 Ludwigshafen (DE); KNÖSCHE, Carsten, 67150 Niederkirchen (DE)
(86) Internationale Anmeldenummer: PCT/EP2002/012930
(87) Internationale Veröffentlichungsnummer: WO 2003/045900

(56) Entgegenhaltungen:
- EP-A- 0 289 840
- EP-A- 0 570 799

## Beschreibung

Die Erfindung betrifft ein Verfahren zur Herstellung von Isocyanaten durch Umsetzung von primären Aminen mit Phosgen in der Gasphase, **dadurch gekennzeichnet, dass** die Umsetzung von Amin und Phosgen in einem Reaktionskanal, bevorzugt einem Plattenreaktor erfolgt, wobei die Innenabmessungen des Reaktionskanals ein Verhältnis von Breite zu Höhe von mindestens 2 : 1 aufweisen.

Es sind aus dem Stand der Technik verschiedene Verfahren zur Herstellung von Isocyanaten durch Umsetzung von Aminen mit Phosgen in der Gasphase bekannt. EP-A-593 334 beschreibt ein Verfahren zur Herstellung von aromatischen Diisocyanaten in der Gasphase, **dadurch gekennzeichnet, dass** die Umsetzung des Diamins mit Phosgen in einem Rohrreaktor ohne bewegte Teile und mit einer Verengung der Wände entlang der Längsachse des Rohrreaktors stattfindet. Das Verfahren ist jedoch problematisch, da die Vermischung der Eduktströme allein über eine Verengung der Rohrwand schlecht im Vergleich zur Anwendung eines richtigen Mischorganes funktioniert. Eine schlechte Vermischung führt üblicherweise zu einer unerwünscht hohen Feststoffbildung.

EP-A-699 657 beschreibt ein Verfahren zur Herstellung von aromatischen Diisocyanaten in der Gasphase, **dadurch gekennzeichnet, dass** die Umsetzung des zugehörigen Diamins mit dem Phosgen in einem zweizonigen Reaktor stattfindet, wobei die erste Zone, die 20 % bis 80 % des Gesamtreaktorvolumens ausmacht, ideal vermischt ist und die zweite Zone, die 80 % bis 20 % des Gesamtreaktorvolumens ausmacht, durch eine Kolbenströmung charakterisiert werden kann. Weil jedoch mindestens 20 % des Reaktionsvolumens ideal rückvermischt sind, resultiert eine ungleichmäßige Verweilzeitverteilung, die zu einer unerwünscht erhöhten Feststoffbildung führen kann.

EP-A-289 840 beschreibt die Herstellung von Diisocyanaten durch Gasphasenphosgenierung, wobei die Herstellung erfindungsgemäß in einer turbulenten Strömung bei Temperaturen zwischen 200°C und 600°C in einem zylindrischen Raum ohne bewegte Teile stattfindet. Durch den Verzicht auf bewegte Teile wird die Gefahr eines Phosgenaustrittes reduziert. Durch die turbulente Strömung im zylindrischen Raum (Rohr) wird, wenn man von Fluidelementen in Wandnähe absieht, eine.relative gute Strömungsgleichverteilung im Rohr und damit eine relativ enge Verweilzeitverteilung erreicht, die, wie in EP-A-570 799 beschrieben, zu einer Verringerung der Feststoffbildung führen kann.

EP-A-570 799 betrifft ein Verfahren zur Herstellung von aromatischen Diisocyanaten in der Gasphase, **dadurch gekennzeichnet, dass** die Umsetzung des zugehörigen Diamins mit dem Phosgen in einem Rohrreaktor oberhalb der Siedetemperatur des Diamins innerhalb einer mittleren Kontaktzeit von 0,5 bis 5 Sekunden durchgeführt wird. Wie in der Schrift beschrieben ist, führen zu lange als auch zu kurze Reaktionszeiten zu einer unerwünschten Feststoffbildung. Es wird daher ein Verfahren offenbart, bei dem die mittlere Abweichung von der mittleren Kontaktzeit weniger als 6 % beträgt. Die Einhaltung dieser Kontaktzeit wird dadurch erreicht, dass die Reaktion in einer Rohrströmung durchgeführt wird, die entweder durch eine Reynolds-Zahl von oberhalb 4.000 oder eine Bodenstein-Zahl von oberhalb 100 charakterisiert wird.

EP-A-749 958 beschreibt ein Verfahren zur Herstellung von Triisocyanaten durch Gasphasenphosgenierung von (cyclo)aliphatischen Triaminen mit drei primären Aminogruppen, **dadurch gekennzeichnet, dass** man das Triamin und das Phosgen kontinuierlich in einem auf 200°C bis 600°C erhitzten, zylindrischen Reaktionsraum mit einer Strömungsgeschwindigkeit von mindestens 3 m/s miteinander zur Reaktion bringt.

EP-A-928785 beschreibt die Verwendung von Mikrostrukturmischern zur Phosgenierung von Aminen in der Gasphase. Nachteilig bei der Verwendung von Mikromischern ist, dass bereits kleinste Feststoffmengen, deren Entstehung bei der Synthese der Isocyanate nicht vollständig auszuschließen ist, zum Verstopfen des Mischers führen können, was die zeitliche Verfügbarkeit der Phosgenierungsanlage herabsetzt.

Für die Herstellung von Isocyanaten im großtechnischen Maßstab bieten sich bei den bekannten Gasphasenphosgenierungsverfahren, die einen zylindrischen Reaktionsraum verwenden, zwei Möglichkeiten der technischen Realisierung an. Zum einen kann die Reaktion in einer einzigen Rohrstrecke durchgeführt werden, deren Durchmesser an die Produktionskapazität der Anlage angepasst werden muss. Für sehr große Produktionsanlagen hat dieses Konzept den Nachteil, dass eine genaue Temperierung der Reaktionsströme (Phosgen und Amin) im Kern der Strömung durch eine Wandheizung des Rohres nicht mehr realisierbar ist. Lokale Temperaturinhomogenitäten können aber bei zu hoher Temperatur zur Produktzersetzung oder bei zu niedriger Temperatur zur ungenügenden Umsetzung der Edukte zum gewünschten Isocyanat führen.

Die zweite Möglichkeit der technischen Realisierung ist die Aufteilung des reagierenden Gemisches in einzelne Teilströme, die dann parallel durch kleinere, einzelne Rohre geleitet werden, die aufgrund ihres kleineren Durchmessers besser temperierbar sind. Nachteilig bei dieser Verfahrensvariante ist, dass diese Variante relativ verstopfungsanfällig ist, wenn nicht der Volumenstrom durch jedes einzelne Rohr geregelt wird. Bekommt z.B. eines der Rohre an einer Stelle Ablagerungen, dann ist der Druckverlust der Strömung durch dieses Rohr größer. Das Reaktionsgas weicht deshalb automatisch verstärkt auf die anderen Rohre aus. Das Rohr mit den Ablagerungen wird weniger durchströmt. Wenn aber die Strömung in dem sich zu Verstopfen beginnenden Rohr weniger wird, hat die Strömung in diesem Rohr eine verlängerte Verweilzeit, was wie bereits in EP 570 799 erklärt zur Erhöhung der Feststoffbildung führt. Außerdem können sich eventuelle Feststoffe aus einer langsamen Strömung wesentlich leichter an der Rohrwand absetzen, was das Verstopfen beschleunigt.

Zusammenfassend gesagt hat bei der großtechnischen Gasphasenphosgenierung die Verwendung eines großen Rohres das Problem der Temperierung der Gesamtströmung und die Verwendung vieler kleiner Rohre die Gefahr der ungleichmäßigen Durchströmung. Beide Problemfelder führen zu einem ungleichmäßigen Reaktionsfortschritt und damit zu Feststoffen, was die Betreibbarkeit der Anlage herabsetzt.

Aufgabe der Erfindung ist es daher, ein Verfahren zur Herstellung von Isocyanaten durch Phosgenierung in der Gasphase bereitzustellen, wobei sowohl eine hohe Wärmeaustauschfläche als auch trotz nicht vollständig zu verhindernde Feststoffbildung eine möglichst lange Betriebszeit der Produktionsanlage, insbesondere einer großtechnischen Produktionsanlage, erreicht wird.

Überraschenderweise wurde gefunden, dass die kontinuierliche Phosgenierung von Aminen in der Gasphase vorteilhaft, d.h. beispielsweise bei wesentlich erhöhter Betriebsstundenzahl der Produktionsanlage, durchgeführt werden kann, wenn die Reaktion in einem nicht zylindrischen Reaktionskanal, bevorzugt einem Plattenreaktor, durchgeführt wird, der bevorzugt eine Höhe aufweist, welche eine vorteilhafte Temperierung der Reaktanten ermöglicht und der eine Breite aufweist, die mindestens das zweifach der Höhe beträgt.

Gegenstand der Erfindung ist daher ein Verfahren zur Herstellung von Isocyanaten durch Umsetzung von primären Aminen mit Phosgen in der Gasphase, **dadurch gekennzeichnet, dass** die Umsetzung von Amin und Phosgen in einem Reaktionskanals erfolgt, wobei die Innenabmessungen des Reaktionskanals ein Verhältnis von Breite zu Höhe von mindestens 2 : 1 aufweisen.

Ferner ist Gegenstand der Erfindung die Verwendung eines Plattenreaktors, wobei die Innenabmessungen des Plattenreaktors ein Verhältnis von Breite zu Höhe von mindestens 2 : 1 aufweisen, zur Herstellung von Isocyanaten durch Umsetzung von primären Aminen mit Phosgen in der Gasphase.

Schließlich ist Gegenstand der Erfindung die Verwendung eines Reaktorblocks, enthaltend mindestens zwei der vorstehend beschriebenen Plattenreaktoren, zur Herstellung von Isocyanaten durch Umsetzung von primären Aminen mit Phosgen in der Gasphase.

Der in der vorliegenden Erfindung verwendete Reaktionskanal besteht im allgemeinen aus einem Material, dass im wesentlichen inert gegenüber der Phosgenierungsreaktion ist. Ferner sollte es im allgemeinen Drücke bis zu 10 bar, bevorzugt bis zu 20 bar standhalten. Geeignete Materialien sind z.B. Metalle, wie Stahl, Silber oder Kupfer, Glas, Keramik oder homogenen oder heterogenen Gemischen daraus. Bevorzugt werden Stahlreaktoren verwendet.

Der verwendete Reaktionskanal ist bevorzugt im wesentlichen quaderförmig. Der verwendete Reaktionskanal weist ein Verhältnis von Breite zu Höhe von mindestens 2 : 1, bevorzugt mindestens 3 : 1, besonders bevorzugt mindestens 5 : 1 und insbesondere mindestens 10 : 1 auf. Die obere Grenze des Verhältnisses von Breite zu Höhe hängt von der gewünschten Kapazität des Reaktionskanals ab und ist prinzipiell nicht begrenzt. Technisch sinnvoll haben sich Reaktionskanäle mit einem Verhältnis von Breite zu Höhe bis maximal 5000 : 1, bevorzugt 1000 : 1 erwiesen.

Die Höhe des Reaktionskanals ist im allgemeinen nicht beschränkt. So ist es beispielsweise möglich, die Umsetzung in einem hohem Reaktionskanal mit einer Höhe von beispielsweise 40 cm durchzuführen. Sofern jedoch ein besserer Wärmeaustausch mit den Reaktorwänden erfolgen soll, kann die Umsetzung in Reaktionskanälen mit geringer Höhe, beispielsweise nur einige Zentimeter oder Millimeter, durchgeführt werden.

Im allgemeinen weist der Reaktionskanal eine Höhe von 1 Millimeter bis 50 Zentimeter auf, bevorzugt von 2 Millimeter bis 20 Zentimeter, besonders bevorzugt von 3 Millimeter bis 3 Zentimeter.

Die Länge des Reaktionskanals ist abhängig vom gewünschten Umsatzgrad der Umsetzung und beträgt im allgemeinen das Zehnfache der Höhe des Reaktionskanals, bevorzugt das Zwanzigfache, insbesondere das Fünfzigfache der Höhe des Reaktionskanals. Die Länge des Reaktors überschreitet bevorzugt nicht das 10000fache, besonders bevorzugt nicht das 5000fache, insbesondere nicht das 4000fache, der Höhe des Reaktionskanals.

In einer bevorzugten Ausführungsform beträgt die Länge des Reaktionskanals mindestens 1 Meter, üblicherweise 2 bis 50 Meter, bevorzugt 5 bis 25 Meter, besonders bevorzugt 10 bis 20 Meter.

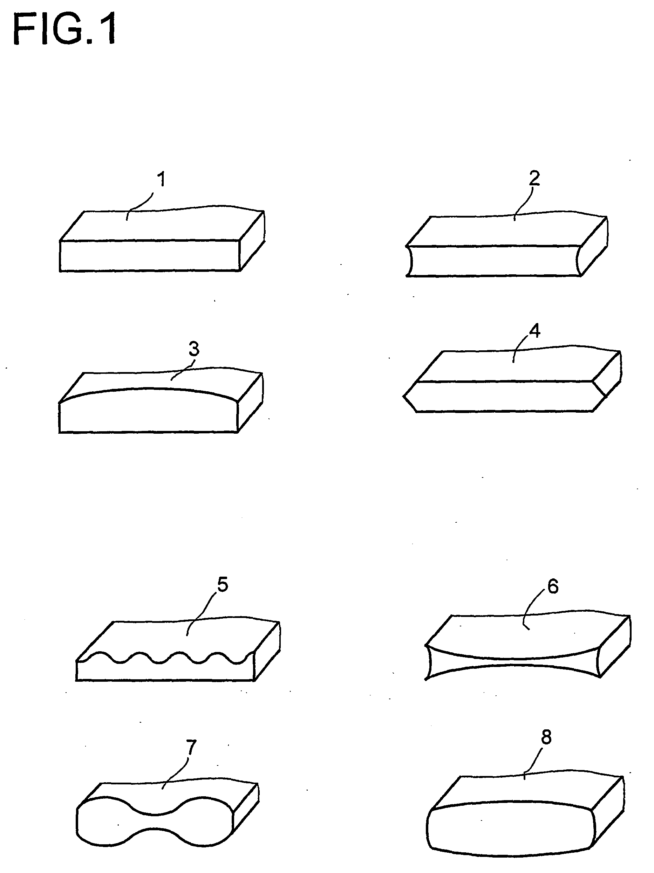

Der verwendete Reaktionskanal ist bevorzugt im wesentlichen quaderförmig. Je nach technischer Ausgestaltung kann er jedoch abgerundete Ecken und/oder Kanten aufweisen. Ebenfalls kann die den Quader bildende Flächen nicht plan sondern gebogen sein. Sofern die Höhe, Breite oder Länge nicht an allen Stellen des Quaders gleich ist, so betreffen die in der Beschreibung und in den Ansprüchen offenbarten Werte für die Innenabmessungen des Reaktionskanals die mittlere (d.h. durchschnittliche) Höhe, mittlere Breite oder mittlere Länge. Mögliche Ausführungsformen des Reaktionskanals zeigen die Abbildungen 1 bis 8 in Figur 1.

Die Wände des Reaktionskanals können glatt oder profiliert sein. Als Profile eignen sich beispielsweise Ritzen oder Wellen.

Bei dem in der vorliegenden Erfindung verwendeten Reaktionskanal handelt es sich bevorzugt um einen Plattenreaktor. Dieser ist bevorzugt aufgebaut aus plattenförmigen Schichten. Besonders bevorzugt werden rechteckige, plattenförmige Schichten verwendet. Im allgemeinen kann der Plattenreaktor aus vier rechteckigen, plattenförmigen Materialien zusammengesetzt werden, so dass der Plattenreaktor bevorzugt die Form eines Quaders aufweist. Die Kanten eines derartigen Quaders können eine Winkel von etwa 90 ° aufweisen, sie können hingegen jedoch auch abgerundet sein.

Ebenfalls ist es möglich, den Plattenreaktor aus zwei plattenförmigen Schicht mit mindestens einer zu etwa 90 ° aufgebogenen Kante zusammen zu setzten, so dass ein quaderförmiger Reaktionskanal gebildet wird.

In einer bevorzugten Ausführungsform enthält der Reaktionskanal an der Eintrittsöffnung ein Verteilelement, wobei das Verteilelement geeignete Umlenkelemente enthält, die den Gasstrom im wesentlichen über die gesamte Breite des Reaktionskanals möglichst homogen verteilen. Die genannten Umlenkelemente können beispielsweise aus gebogenen Blechen bestehen.

Die Vermischung der Reaktionskomponenten Amin und Phosgen kann vor oder im Reaktionskanal erfolgen. So ist es möglich, dem Reaktionskanal eine Mischeinheit, beispielsweise eine Düse, vorzuschalten, wodurch bereits ein gemischter Gasstrom, enthaltend Phosgen und Amin, in den Reaktionskanal gelangt.

In einer bevorzugten Ausführungsform wird zunächst der Phosgenstrom mittels eines Verteilelements auf die gesamte Breite des Reaktionskanals möglichst homogen verteilt. Die Zufuhr des Aminstroms erfolgt am Anfang der Reaktionskanals, hier ist ein Verteilerkanal mit Löchern oder Mischdüsen im Reaktionskanal eingebracht, wobei dieser Verteilerkanal bevorzugt über die gesamte Breite des Reaktionskanals reicht. Aus den Löchern oder Mischdüsen wird das Amin, das gegebenenfalls mit einem Inertmedium vermischt ist, dem Phosgenstrom zugeführt.

Bei dem Inertmedium handelt es sich um ein Medium, das bei der Reaktionstemperatur gasförmig vorliegt und nicht mit den Edukten reagiert. Beispielsweise können Stickstoff, Edelgase wie Helium oder Argon oder Aromaten wie Chlorbenzol, Dichlorbenzol oder Xylol verwendet werden. Bevorzugt wird Stickstoff als Inertmedium verwendet.

Für das erfindungsgemäße Verfahren können primäre Amine verwendet werden, die bevorzugt ohne Zersetzung in die Gasphase überführt werden können. Besonders geeignet sind hier Amine, insbesondere Diamine, auf Basis von aliphatischen oder cycloaliphatischen Kohlenwasserstoffen mit 1 bis 15 Kohlenstoffatome. Beispiele hierfür sind 1,6-Diaminohexan, 1-Amino-3,3,5-trimethyl-5-amino-methylcyclohexan (IPDA) und 4,4'-Diaminodicyclohexylmethan. Bevorzugt verwendet wird 1,6-Diaminohexan (HDA).

Ebenfalls können für das erfindungsgemäße Verfahren aromatische Amine verwendet werden, die bevorzugt ohne Zersetzung in die Gasphase überführt werden können. Beispiele für bevorzugte aromatische Amine sind Toluylendiamin (TDA), als 2,4- oder 2,6-Isomer oder als Gemisch davon, Diaminobenzol, Napthyldiamin (NDA) und 2,4'- oder 4,4'-Methylen (diphenylamin) (MDA) oder Isomerengemische davon.

Beim erfindungsgemäßen Verfahren ist es vorteilhaft, Phosgen im Überschuss bezüglich Aminogruppen einzusetzen. Üblicherweise liegt ein molares Verhältnis von Phosgen zu Aminogruppen von 1,1 :1 bis 20 : 1, bevorzugt von 1,2 :1 bis 5 :1 vor.

Zur Durchführung des erfindungsgemäßen Verfahrens kann es vorteilhaft sein, die Ströme der Reaktanten vor dem Vermischen vorzuwärmen, üblicherweise auf Temperaturen von 100 bis 600°C, bevorzugt von 200 bis 500°C. Die Umsetzung im Reaktionskanal findet üblicherweise bei einer Temperatur von 150 bis 600°C, bevorzugt von 250 bis 500°C statt. Das erfindungsgemäße Verfahren wird bevorzugt kontinuierlich durchgeführt.

In einer bevorzugten Ausführungsform werden die Abmessungen des Reaktionskanals und die Strömungsgeschwindigkeiten so bemessen, dass eine turbulente Strömung, d.h. eine Strömung mit einer Reynolds-Zahl von mindestens 2300, bevorzugt mindestens 2700, vorliegt, wobei die Reynolds-Zahl mit dem hydraulischen Durchmesser des Reaktionskanales gebildet wird. Bevorzugt durchlaufen die gasförmigen Reaktionspartner den Reaktionskanal mit einer Strömungsgeschwindigkeit von 20 bis 150 Meter/Sekunde, bevorzugt von 30 bis 100 Meter/Sekunde.

Im allgemeinen beträgt bei dem erfindungsgemäßen Verfahren die mittlere Kontaktzeit 0,05 bis 5 Sekunden, bevorzugt von 0,06 bis 1, besonders bevorzugt 0,1 bis 0,45 Sekunden. Unter mittlerer Kontaktzeit wird die Zeitspanne von Beginn der Vermischung der Edukte bis zum Auswaschen des Reaktionsgases nach Verlassen des Reaktionskanals in der Aufarbeitungsstufe verstanden. In einer bevorzugten Ausführungsform ist die Strömung im erfindungsgemäßen Verfahren durch eine Bodenstein-Zahl von mehr als 10, bevorzugt mehr als 100 und besonders bevorzugt von mehr als 500 charakterisiert.

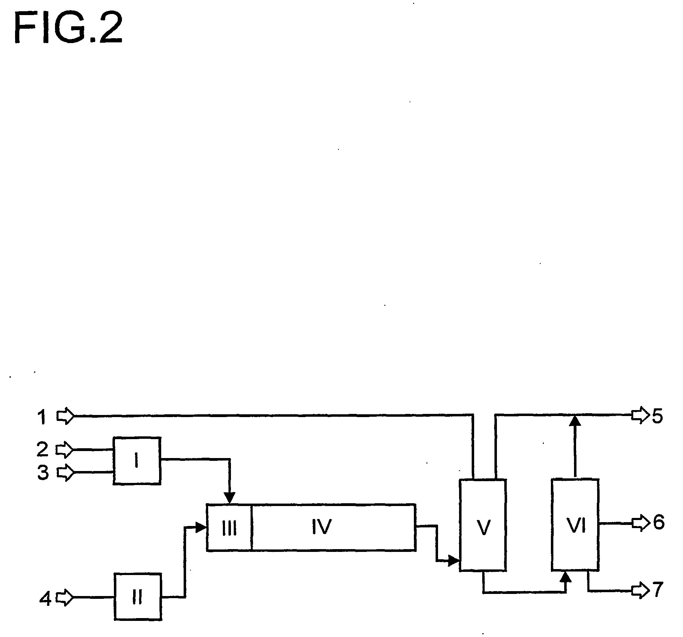

Eine bevorzugte Ausführungsform des erfindungsgemäßen Verfahrens ist in Figur 2 schematisch veranschaulicht.

In Figur 2 bedeutet:
I Aminvorlage
II Phosgenvorlage
III Mischeinheit
IV Reaktionskanal
V Aufarbeitungsstufe
VI Reinigungsstufe
   1 Zufuhr Lösungsmittel
   2 Zufuhr Amin
   3 Zufuhr Inertmedium
   4 Zufuhr Phosgen
   5 Austrag Chlorwasserstoff, Phosgen und/oder Inertmedium
   6 Austrag Lösungsmittel
   7 Austrag Isocyanat

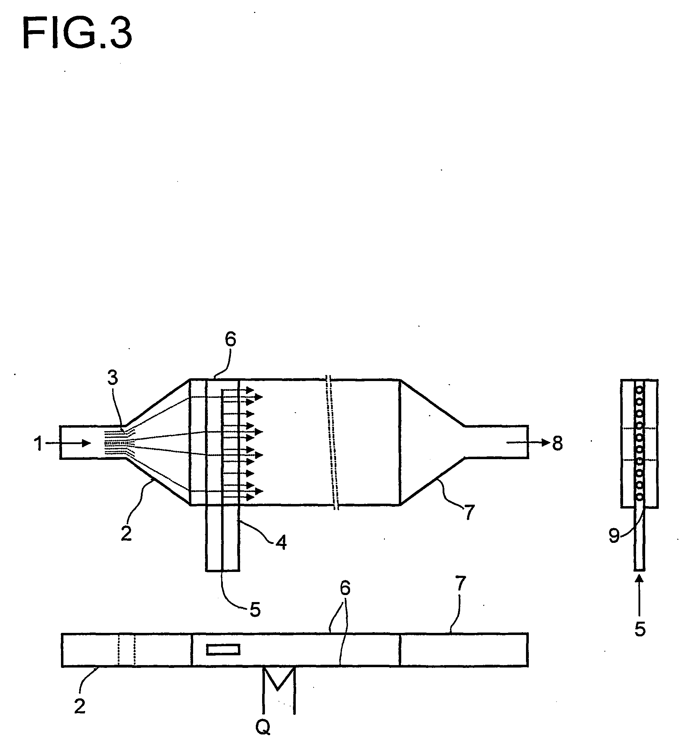

In der Aminvorlage wird das Amin, gegebenenfalls zusammen mit einem Inertmedium als Trägergas wie beispielsweise Stickstoff, in die Gasphase überführt und in die Mischeinheit eingespeist. Ebenfalls wird Phosgen aus der Phosgenvorlage in die Mischeinheit überführt. Nach dem Vermischen in der Mischeinheit, die beispielsweise aus einer Düse oder einem statischen Mischer bestehen kann, wird das gasförmige Gemisch aus Phosgen, Amin und gegebenenfalls Inertmedium in den Reaktionskanal überführt. Wie in Figur 2 veranschaulicht muss es sich bei der Mischeinheit nicht um eine eigenständige Reaktionsstufe handeln, vielmehr kann es vorteilhaft sein, die Mischeinheit in den Reaktionskanal zu integrieren. Eine bevorzugte Ausführungsform einer integrierten Einheit aus Mischeinheit und Reaktionskanal wird in Figur 3 dargestellt.

Nachdem das Reaktionsgemisch im Reaktionskanal umgesetzt wurde, gelangt es in die Aufarbeitungsstufe. Bevorzugt handelt es sich hier um einen sogenannten Waschturm, wobei aus dem gasförmigen Gemisch das gebildete Isocyanat durch Kondensation in einem inerten Lösungsmittel abgetrennt wird, während überschüssiges Phosgen, Chlorwasserstoff und gegebenenfalls das Inertmedium die Aufarbeitungsstufe gasförmig durchlaufen. Als inertes Lösungsmittel sind bevorzugt aromatische Kohlenwasserstoffe, die gegebenenfalls mit Halogenatomen substituiert sind, geeignet, wie beispielsweise Chlorbenzol, Dichlorbenzol, und Toluol. Besonders bevorzugt wird dabei die Temperatur des inerten Lösungsmittel oberhalb der Zersetzungstemperatur des zum Amin gehörigen Carbamoylchlorids gehalten.

In der anschließenden Reinigungsstufe wird das Isocyanat, bevorzugt durch Destillation, vom Lösungsmittel abgetrennt. Ebenfalls kann hier noch die Abtrennung von restlichen Verunreinigungen umfassend Chlorwasserstoff, Inertmedium und/oder Phosgen erfolgen.

Figur 3 veranschaulicht eine bevorzugte Ausführungsform eines Reaktionskanals. In dieser Ausführungsform besteht der Reaktionskanal aus einem Plattenreaktor, der an den beiden Austrittsöffnungen ein Verteilelement und ein Sammelelement enthält.

In Figur 3 bedeutet:
- 1: Zufuhr Phosgenstrom
- 2: Verteilelement
- 3: Umlenkelemente
- 4: Verteilerkanal mit Austrittsöffnungen für Amin
- 5: Aminzufuhr
- 6: Plattenreaktor
- 7: Sammelelement
- 8: Austrittskanal
- 9: Austrittsöffnungen für Amin

Der eingesetzte gasförmige Phosgenstrom wird durch ein Verteilelement auf die gesamte Breite des Plattenreaktors möglichst homogen verteilt. Hierzu können Umlenkelemente verwendet werden, die beispielsweise aus gewinkelten Blechen bestehen können. Die Zugabe des Amins erfolgt über einen Verteilerkanal, der sich bevorzugt über die gesamte Breite des Plattenreaktors erstreckt und bevorzugt in räumlicher Nähe zum Verteilelement angeordnet ist. Der Verteilerkanal ist bevorzugt in mittlere Höhe im Plattenreaktor angeordnet und enthält Löcher oder Mischdüsen, über die der Aminstrom dem Plattenreaktor zugeführt und somit mit dem Phosgenstrom vermischt wird. Am anderen Ende des Plattenreaktors werden der Gasstrom bevorzugt in einem Sammelelement fokussiert und anschließend der Aufarbeitungsstufe zugeführt.

Die Zugabe des inerten Lösungsmittels zum Auswaschen des Isocyanates kann im Waschturm und/oder bereits im Austrittsbereich des Plattenreaktors 6 und/oder zwischen Plattenreaktor 6 und Sammelelement 7 und/oder im Sammelelement 7 und/oder im Austrittskanal 8 erfolgen. Eine vorteilhafte Ausführungsform ist **dadurch gekennzeichnet, dass** die Reaktionsgase aus dem Reaktionskanal möglichst nach gleicher Verweilzeit im Reaktionskanal mit dem inerten Lösungsmittel in Kontakt gebracht werden. In einer bevorzugten Ausführungsform wird das inerte Lösungsmittel mit Düsen mit dem Reaktionsgas in Kontakt gebracht, welche an den oben genannten Stellen angebracht sind.

In einer bevorzugten Ausführungsform wird das erfindungsgemäße Verfahren in einem Reaktorblock durchgeführt, wobei der Reaktorblock zwei oder mehr, bevorzugt 2 bis 20, vorstehend beschriebene Reaktionskanäle, bevorzugt Plattenreaktoren, enthält. Die Reaktionskanäle können innerhalb des Reaktorblocks beliebig angeordnet sein, bevorzugt sind sie übereinander angeordnet. Als Reaktorblock ist beispielsweise eine entsprechend dimensionierte Röhre geeignet, in deren Inneren zwei oder mehr Reaktionskanäle angeordnet sind. Die Umsetzung von Phosgen zu Amin findet folglich lediglich in den erfindungsgemäßen Reaktionskanälen statt, nicht im dem Reaktorblock. Der Reaktorblock dient lediglich als eine Art Mantel für die Reaktionskanäle.

In einer besonders bevorzugten Ausführungsform enthält der Reaktorblock neben den Reaktionskanälen noch zusätzlich ein Fluid, das zwischen den einzelnen Reaktionskanälen fließen kann. Diese Fluid dient zur Wärmeabfuhr, d.h. zur Temperaturregelung innerhalb der Reaktionskanäle. Als Fluid geeignet sind Stoffe, die Temperaturen bis zu etwa 350°C ohne Zersetzung Stand halten, beispielsweise Wärmeträgeröle, wie z.B. Marlotherm^{®}, oder Salzschmelzen, wie z.B. Na/K-Nitrite und/oder Na/K-Nitrate. Ebenfalls ist Wärmeabfuhr durch Siedekühlung, beispielsweise durch Wasser, möglich.

Die Erfindung soll anhand der nachfolgenden Beispiele erläutert werden.

### Beispiel 1: Verwendung eines Plattenreaktors

40 mol/h Hexamethylendiamin und 40 mol/h Monochlorbenzol wurden miteinander vermischt, verdampft und auf 320°C vorgeheizt. Der resultierende Gasstrom wurde mit 160 mol/h gasförmigen Phosgen, das ebenfalls auf eine Temperatur von 320°C vorgeheizt wurde, in einer Mischdüseneinrichtung bestehend aus einer Mischkammer mit zwei gegenüberliegenden Einlässen vermischt, mit einer Eintrittsgeschwindigkeit von jeweils 80 m/s auf Phosgen- und Hexamethylendiamin/Monochlorbenzoleintrittsseite. Das aus der Mischkammer austretende Gasgemisch wurde auf einen Rechteckkanal mit einer Breite von 8 mm und einer Höhe von 62,8 mm und einer Länge von 20 m geleitet. Der Kanal wurde wurden auf den beiden 62,8 mm breiten Seiten auf eine Temperatur von 320°C mit elektrischen Heizbändern temperiert. Nach Austritt aus dem Kanal wurde das Hexamethylendiisocyanat (HDI) selektiv aus dem Reaktionsgemisch in bekannter Art und Weise (siehe z.B. US 2 480 089) oberhalb der Zersetzungstemperatur des zugehörigen Carbamylchlorids mit Monochlorbenzol bei einer Temperatur von 165°C ausgewaschen. Das gewünschte HDI wurde destillativ vom Waschmittel Monochlorbenzol mit einer Ausbeute von 98,5 % abgetrennt. Durch Anlegen eines Unterdruckes an den Waschturm wurden im Waschturm und in den vorgeschalteten Reaktionsrohren ein Absolutdruck von 350 mbar eingestellt. Zwischen dem Kanal und der Mischeinrichtung sowie im Waschturm wurden Druckmessungen eingebaut. Am Anfang des Versuches stellte sich zwischen den Druckmessstellen zwischen Mischer und Kanal und der Druckmessstelle am Eintritt in den Waschturm eine Druckdifferenz von ca. 79 mbar ein, was in etwa dem DruckverIust der Strömung durch Reibung im Rohr entspricht.

Nach 24 h Betriebsdauer war noch kein Anzeichen für einen Anstieg der Druckdifferenz zu erkennen, was ein Anzeichen für die Verengung des freien Querschnitts durch Feststoffbildung im Rohr wäre.

Vergleichsbeispiel 2: Verwendung eines Rohrreaktors bestehend aus 10 parallelen Teilrohren, wobei die Gesamtquerschnittsfläche aller Rohre der Querschnittsfläche zwischen den zwei Platten entspricht.

Analog zu Beispiel 1 wurden 40 mol/h Hexamethylendiamin und 40 mol/h Monochlorbenzol miteinander vermischt, verdampft und auf 320°C vorgeheizt. Der resultierende Gasstrom wurde mit 160 mol/h gasförmigen Phosgen, das ebenfalls auf eine Temperatur von 320°C vorgeheizt wurde, in einer Mischdüseneinrichtung bestehend aus einer Mischkammer mit zwei gegenüberliegenden Einlässen vermischt, mit einer Eintrittsgeschwindigkeit von jeweils 80 m/s auf Phosgen- und Hexamethylendiamin/Monochlorbenzoleintrittsseite. Das aus der Mischkammer austretende Gasgemisch wurde auf 10 parallele Rohre mit einem kreisförmigen Durchmesser von 8 mm und einer Länge von 20 m verteilt. Die Rohre wurden auf eine Temperatur von 320°C mit elektrischen Heizbändern temperiert. Nach Austritt aus den Rohren wurde das Hexamethylendiisocyanat (HDI) selektiv aus dem Reaktionsgemisch in bekannter Art und Weise (siehe z.B. US 2 480 089) oberhalb der Zersetzungstemperatur des zugehörigen Carbamylchlorids mit Monochlorbenzol bei einer Temperatur von 165°C ausgewaschen. Das gewünschte HDI wurde destillativ vom Waschmittel Monochlorbenzol mit einer Ausbeute von 98,5 % abgetrennt. Durch Anlegen eines Unterdruckes an den Waschturm wurde im Waschturm und in den vorgeschalteten Reaktionsrohren ein Absolutdruck von 350 mbar eingestellt. Zwischen den Rohren und der Mischeinrichtung sowie im Waschturm wurden Druckmessungen eingebaut. Am Anfang des Versuches stellte sich zwischen den Druckmessstellen zwischen Mischer und Reaktionsrohr und der Druckmessstelle am Eintritt in den Waschturm eine Druckdifferenz von ca. 162 mbar ein, was in etwa dem Druckverlust der Strömung durch Reibung im Rohr entspricht. Nach 24 h Betriebsdauer stieg die Druckdifferenz in einem der Rohre von 162 mbar auf 187 mbar, was ein Anzeichen für die Verengung des freien Querschnitts durch Feststoffbildung im Rohr ist. Der Versuch wurde daraufhin abgebrochen.

## Patentansprüche

1. Verfahren zur Herstellung von Isocyanaten durch Umsetzung von primären Aminen mit Phosgen in der Gasphase, **dadurch gekennzeichnet, dass** die Umsetzung von Amin und Phosgen in einem Reaktionskanal erfolgt, wobei die Innenabmessungen des Reaktionskanals ein Verhältnis von Breite zu Höhe von mindestens 2 : 1 aufweisen.

2. Verfahren nach Anspruch 1, **dadurch gekennzeichnet, dass** es sich bei dem Reaktionskanal um einen Plattenreaktor handelt, wobei die Innenabmessungen des Plattenreaktors ein Verhältnis von Breite zu Höhe von mindestens 2 : 1 aufweisen.

3. Verfahren nach Anspruch 1 oder 2, **dadurch gekennzeichnet, dass** die Innenabmessungen des Reaktionskanals eine Höhe von 1 Millimeter bis 50 Zentimeter aufweisen.

4. Verfahren nach einem der Ansprüche 1 bis 3, **dadurch gekennzeichnet, dass** die Länge des Reaktionskanals mindestens das Zehnfache der Breite des Reaktionskanals beträgt.

5. Verfahren nach einem der Ansprüche 1 bis 4, **dadurch gekennzeichnet, dass** als primäres Amin Toluylendiamin, 1,6-Diaminohexan, 1-Amino-3,3,5-trimethyl-5-aminomethylcyclohexan oder 4,4'-Diaminodicyclohexylmethan, verwendet wird.

6. Verfahren nach einem der Ansprüche 1 bis 5, **dadurch gekennzeichnet, dass** das eingesetzte gasförmige Phosgen durch ein Verteilelement eingangs des Reaktionskanals über die gesamte Breite des Reaktionskanals verteilt wird und das gasförmige Amin dem verteilten Phosgenstrom zugeführt wird.

7. Verfahren nach einem der Ansprüche 2 bis 6, **dadurch gekennzeichnet, dass** die Umsetzung in einem Reaktorblock, enthaltend zwei oder mehr der in Anspruch 2 beschriebenen Plattenreaktoren, erfolgt.

8. Verfahren nach Anspruch 7, **dadurch gekennzeichnet, dass** der Reaktorblock ein zur Temperaturregelung geeignetes Fluid enthält, welches die Plattenreaktoren mindestens teilweise umhüllt.

9. Verwendung eines Reaktionskanals gemäß einem der Ansprüche 1 bis 4 zur Herstellung von Isocyanaten durch Umsetzung von primären Aminen mit Phosgen in der Gasphase.

10. Verwendung eines Reaktorblocks gemäß Anspruch 7 oder 8 zur Herstellung von Isocyanaten durch Umsetzung von primären Aminen mit Phosgen in der Gasphase.

## Claims

1. A process for the preparation of isocyanates by reacting primary amines with phosgene in the gas phase, wherein the reaction of amine and phosgene is carried out in a reaction channel, the internal dimensions of the reaction channel having a width-to-height ratio of at least 2 : 1.

2. The process according to claim 1, wherein the reaction channel is a plate reactor, the internal dimensions of the plate reactor having a width-to-height ratio of at least 2 : 1.

3. The process according to claim 1 or 2, wherein the internal dimensions of the reaction channel have a height of from 1 millimeter to 50 centimeters.

4. The process according to any of claims 1 to 3, wherein the length of the reaction channel is at least ten times the width of the reaction channel.

5. The process according to any of claims 1 to 4, wherein the primary amine used is toluenediamine, 1,6-diaminohexane, 1-amino-3,3,5-trimethyl-5-aminomethylcyclohexane or 4,4'-diaminodicyclohexylmethane.

6. The process according to any of claims 1 to 5, wherein the gaseous phosgene used is distributed over the total width of the reaction channel by a distributing element at the entrance of the reaction channel and the gaseous amine is fed to the distributed phosgene stream.

7. The process according to any of claims 2 to 6, wherein the reaction is carried out in a reactor block comprising two or more of the plate reactors described in claim 2.

8. The process according to claim 7, wherein the reactor block comprises a fluid which is suitable for temperature regulation and at least partly surrounds the plate reactors.

9. The use of a reaction channel according to any of claims 1 to 4 for the preparation of isocyanates by reacting primary amines with phosgene in the gas phase.

10. The use of a reactor block according to claim 7 or 8 for the preparation of isocyanates by reacting primary amines with phosgene in the gas phase.

## Revendications

1. Procédé pour la production d'isocyanates par la réaction d'amines primaires avec du phosgène en phase gazeuse, **caractérisé en ce que** la réaction de l'amine et du phosgène s'effectue dans un canal de réaction, les dimensions internes du canal de réaction présentant un rapport de la largeur à la hauteur d'au moins 2:1.

2. Procédé selon la revendication 1, **caractérisé en ce que** le canal de réaction consiste en un réacteur à plaques, les dimensions internes du réacteur à plaques présentant un rapport de la largeur à la hauteur d'au moins 2:1.

3. Procédé selon la revendication 1 ou 2, **caractérisé en ce que** les dimensions interne du canal de réaction ont une grandeur de 1 millimètre à 50 centimètres.

4. Procédé selon l'une quelconque des revendications 1 à 3, **caractérisé en ce que** la longueur du canal de réaction représente au moins le décuple de la largeur du canal de réaction.

5. Procédé selon l'une quelconque des revendications 1 à 4, **caractérisé en ce qu'**on utilise comme amine primaire la toluylènediamine, le 1,6-diaminohexane, le 1-amino-3,3,5-triméthyl-5-aminométhyl-cyclohexane ou le 4,4'-diaminodicyclohexylméthane.

6. Procédé selon l'une quelconque des revendications 1 à 5, **caractérisé en ce que** le phosgène gazeux utilisé est réparti sur toute la largeur du canal de réaction par un élément distributeur à l'entée du canal de réaction et l'amine gazeuse est envoyée au flux de phosgène réparti.

7. Procédé selon l'une quelconque des revendications 2 à 6, **caractérisé en ce que** la réaction s'effectue dans un bloc de réacteurs comportant deux ou plus de deux réacteurs à plaques décrits dans la revendication 2.

8. Procédé selon la revendication 7, **caractérisé en ce que** le bloc de réacteurs contient un fluide approprié à la régulation de la température, qui enveloppe au moins en partie les réacteurs à plaques.

9. Utilisation d'un canal de réaction selon l'une quelconque des revendications 1 à 4, pour la production d'isocyanates par réaction d'amines primaires avec du phosgène en phase gazeuse.

10. Utilisation d'un bloc de réacteurs selon la revendication 7 ou 8, pour la production d'isocyanates par réaction d'amines primaires avec du phosgène en phase gazeuse.
